# NOUVEAU FASCICULE DE BREVET EUROPEEN

(11) **EP 0 363 777 B2**
(45) Date de publication et mention de la décision concernant l'opposition: **16.04.1997**
(45) Mention de la délivrance du brevet: 08.04.1992
(21) Numéro de dépôt: 89118236.2
(22) Date de dépôt: 02.10.1989
(51) Int. Cl.: A61K 35/78

(54) **Procédé de préparation d'extrait en poudre de racines de valériane**
Verfahren zur Herstellung eines Pulverextraktes aus Baldrianwurzeln
Process for the preparation of a powder extract from valerian roots

(30) Priorité: 12.10.1988 EP 88116920
(43) Date de publication de la demande: 18.04.1990
(73) Titulaire: SOCIETE DES PRODUITS NESTLE S.A., 1800 Vevey (CH)
(72) Inventeur: Cerise, Léon, CH-1807 Blonay (CH); Leathwood, Peter D., CH-1807 Blonay (CH); Lunder, Tito Livio, CH-1110 Morges (CH)

(56) Documents cités:
- EP-A- 0 011 718
- EP-B- 0 099 557
- FR-M- 5 640
- H. BRAUN: "Heilpflanzen-Lexikon für Ärzte und Apotheker", 1974, pages 194-195, Gustav Fischer Verlag, Stuttgart
- E. Dieterich "Neues Pharmazeutisches Manual", 11. Auflage, 1913, J. Springer Verlag, Berlin, p. 137-138
- H. Finzelberg's Nachfolger "Hermes Nr. 6" Herstellungsanweisung, 08.05.1985

## Description

L'invention concerne un procédé de préparation d'extrait en poudre désodorisé de racines de valériane.

On connaît l'extrait aqueux de racine de valériane quant à sa propriété d'améliorer le sommeil chez l'être humain (Pharmacology Biochemistry & Behavior, Vol. 17, pages 65 à 71). Ces mélanges sont consommés comme infusions et le mauvais goût et la mauvaise odeur de ces boissons est connue. D'autre part, la poudre de valériane connue actuellement, lorsqu'elle est dissoute dans l'eau, conduit à certains dépôts et à une boisson turbide. L'article de "Neues Pharmazeutisches Manual", Eugen Dieterich, Berlin 1913, pages 137-138, concerne un procédé d'extraction à l'eau de diverses plantes, mentionnant la possibilité de précipiter avec un alcool les substances responsables de la turbidité, mais sans préciser qu'il peut s'agir de valériane et sans indiquer les températures d'extraction à l'eau. Dans les instructions de fabrication du produit No. 0628100 de H. Finzelberg's Nachfolger du 8 mai 1985, il est question d'un procédé d'obtention d'une poudre à base de valériane, mais dans lequel il n'est pas mentionné la possibilité de précipiter les produits responsables de la turbidité, ni une température d'extraction à l'eau permettant d'obtenir une extraction satisfaisante. Le but de la présente invention est donc de mettre au point un procédé de préparation d'extrait en poudre de racines de valériane dans lequel on désodorise la valériane, on élimine les substances qui sont responsables de la turbidité dans la boisson de valériane reconstituée et on obtient une extraction satisfaisante de la valériane.

L'invention concerne un procédé de préparation d'extrait en poudre de racines de valériane, dans lequel on extrait au moins une fois avec de l'eau les racines de valériane broyées à une température comprise entre 65 et 75°C pendant entre 2 et 5 heures, on concentre l'extrait aqueux à une pression comprise entre 2.10⁴ Pa et 2.10³ Pa à une température comprise entre 30 et 60°C. pour arriver à une teneur en matières sèches comprise entre 10 et 20%, on ajoute de l'alcool ou de l'acétone au concentré pour faire précipiter les substances gélatineuses responsables de l'aspect trouble de la boisson reconstituée à partir de la poudre, on ajoute de la malto-dextrine au mélange hydro-alcoolique ou hydro-acétonique centrifugé et on sèche pour obtenir une poudre instantanée.

Par valériane, on entend une plante herbacée vivace du genre Valeriana, que l'on trouve dans les régions tempérées d'Amérique du Nord, d'Europe et d'Asie. Parmi les 170 espèces connues, c'est la valériane commune (Valeriana officinalis L.) qui est le plus souvent utilisée à des fins médicinales. Il est bien entendu que le procédé selon l'invention peut être mis en oeuvre sur toutes les espèces de valériane. Par racines de valériane, on entend la partie souterraine de la plante de valériane.

L'extraction à l'eau est faite à une température comprise entre 65° et 75°C pendant une durée comprise entre 2 et 5 heures. La température de l'eau est critique car une température trop élevée aurait tendance à dégrader certains composants de la racine et une température trop faible ne donne pas une extraction satisfaisante. On effectue de préférence trois extractions successives à l'eau. Cette extraction assure la dégradation des valépotriates tout en préservant les acides valéréniques des racines de valériane.

On concentre ensuite l'extrait aqueux pour en éliminer les odeurs désagréables qui sont volatiles, lesdites odeurs étant produites par des produits de dégradation. Cette concentration se fait à une température comprise entre 30° et 60°C à une pression de l'ordre de 2.10⁴ Pa. Au bout d'une heure environ les substances odoriférantes étant chassées, on baisse la pression à environ 2.10³ Pa pour finir l'élimination d'eau jusqu'à une teneur en matières sèches d'environ 13 à 17%. Après cette étape de concentration, il reste à effectuer le traitement à l'alcool ou à l'acétone. L'alcool utilisé est de l'éthanol. On mélange de manière à arriver à une concentration en alcool ou en acétone comprise entre 48 et 52%. On laisse reposer à température ambiante pendant entre 8 et 20 heures de manière à ce que les composés gélatineux insolubles précipitent. Le précipité est séparé par centrifugation et le reste de l'extrait aqueux est mélangé avec une malto-dextrine avant le séchage.

La fonction de cette malto-dextrine est de servir de support pour le séchage. Elle permet en outre d'obtenir une poudre finale qui n'est pas trop hygroscopique et ayant une bonne durée de conservation.

On effectue alors le séchage soit sous vide soit par pulvérisation pour obtenir une poudre de valériane instantanée qui a conservé son caractère sédatif.

Si on reconstitue la boisson dans l'eau, elle est exempte de dépôt et de turbidité, elle a un goût neutre et aucune odeur. L'absence de turbidité a été mise en évidence par des mesures néphélométriques. La poudre de valériane reconstituée dans l'eau donne un pic de densité optique maximum à environ 650 nm. On constate en outre qu'il n'y a pas de valépotriate dans cette poudre.

Dans le procédé selon l'invention, l'alcool ou l'acétone permettent de séparer les composés responsables de la turbidité. Une autre fonction du traitement à l'alcool est de garantir un procédé presque stérile dans lequel les risques de contaminations bactériennes sont minimalisés.

On arrive à un rendement d'extraction compris entre 20 et 30 %.

Le procédé selon l'invention est mis en oeuvre par charges ou par percolation.

La suite de la description est faite en référence aux exemples.

### Exemple 1

Un percolateur ayant une capacité de 2000 litres est rempli avec 250 kg de racines de valériane finement broyées et 1500 kg d'eau préchauffée à 70°C. Un tamis au fond du percolateur retient les particules et un manteau chauffant maintient la température à 70°C pendant les 4 heures de la première extraction. L'extrait est recyclé de manière continue à partir du fond du récipient d'extraction grâce à une pompe.

Le premier extrait est pompé hors du percolateur vers un réservoir d'où il est envoyé immédiatement vers l'évaporateur sous vide ayant un manteau chauffant dans lequel circule de l'eau à 48°C. C'est dans cet évaporateur qu'a lieu l'élimination des substances volatiles en appliquant pendant une heure un vide de 10⁴ Pa et lorsqu'on arrive à une teneur en matières sèches de 14% on applique un vide de 2.10³ Pa.

On ajoute ensuite de l'alcool à 94% (éthanol) à l'extrait concentré de manière à arriver à une teneur en éthanol de 50%. On laisse reposer pour que le précipité se dépose.

Une seconde extraction de trois heures est effectuée dans le même percolateur avec 1000 kg d'eau fraîche. Une troisième extraction est effectuée dans le même percolateur pendant deux heures. Les second et troisième extraits sont traités comme le premier.

Le réservoir contient, à la fin des trois extractions, l'extrait total contenant 50% d'alcool. La précipitation complète des solides insolubles nécessite une nuit (12 heures). Une centrifugeuse est utilisée pour séparer l'extrait liquide clair des solides précipités. On détermine la teneur en matières sèches de l'extrait centrifugé pour calculer la quantité de malto-dextrine à ajouter de manière à obtenir une dilution de 1:1 en poids.

La quantité calculée de malto-dextrine est introduite dans un séchoir sous vide et l'extrait alcoolique clair et concentré y est ajouté. On chasse l'alcool à 50°C sous une pression réduite atteignant progressivement 10³ Pa.

Le séchage final est effectué dans le même séchoir ou, lorsque l'extrait a atteint la consistance d'un sirop, celui-ci est séché par pulvérisation.

On arrive à un rendement final de poudre de valériane compris entre 23 et 25 %, c'est-à-dire qu'à partir des 250 kg de racines de valériane, on obtient environ 62 kg de poudre de valériane.

### Exemple 2

On répète l'exemple 1, mais au lieu d'utiliser de l'éthanol, on utilise de l'acétone.

On arrive à un rendement d'extraction équivalent à celui avec l'alcool.

La différence réside dans le fait que la mise en oeuvre du procédé est plus économique.

## Revendications

1. Procédé de préparation d'extrait en poudre désodorisé de racines de valériane caractérisé en ce qu'on extrait au moins une fois avec de l'eau les racines de valériane broyées à une température comprise entre 65 et 75°C pendant entre 2 et 5 heures, on concentre l'extrait aqueux à une pression comprise entre 2.10⁴ Pa et 2.10³ Pa à une température comprise entre 30 et 60°C pour arriver à une teneur en matières sèches comprise entre 10 et 20%, on ajoute de l'alcool ou de l'acétone au concentré pour faire précipiter les substances gélatineuses responsables de l'aspect trouble de la boisson reconstituée à partir de la poudre, on ajoute de la malto-dextrine au mélange hydro-alcoolique ou hydro-acétonique centrifugé et on sèche pour obtenir une poudre instantanée.

2. Procédé selon la revendication 1, caractérisé en ce qu'on fait trois extractions à l'eau.

3. Procédé selon l'une des revendications 1 ou 2, caractérisé en ce qu'on ajoute de l'éthanol ou de l'acétone au concentré pour arriver à une teneur en alcool ou en acétone comprise entre 48 et 52% et on laisse reposer pendant entre 8 et 20 heures à température ambiante.

4. Procédé selon l'une des revendications 1 à 3, caractérisé en ce qu'on sèche sous vide ou par pulvérisation.

5. Procédé selon la revendication 1, caractérisé en ce qu'on extrait trois fois les racines de valériane broyées à une température de 70°C, on concentre l'extrait aqueux à une température de 48°C sous un vide de 10⁴ Pa puis de 2.10³ de manière à obtenir une teneur en matière sèche d'environ 14%, on ajoute de l'éthanol ou de l'acétone de manière à avoir une teneur en alcool ou acétone de 50%, on laisse reposer à température ambiante pendant 12 heures, on centrifuge, on ajoute au mélange hydro-alcoolique ou hydro-acétonique une quantité de malto-dextrine de manière à obtenir un rapport en poids 1:1 matière sèche du mélange hydro-alcoolique ou hydro-acétonique sur malto-dextrine et on sèche à 50°C sous un vide de l'ordre de 10³ Pa.

## Claims

1. A process for the preparation of a deodorised powder form extract of valerian roots, characterised in that the ground valerian roots are extracted at least once with water at a temperature of 65 to 75°C for 2 to 5 hours, the aqueous extract is concentrated under a pressure of from 2.10⁴ Pa to 2.10³ Pa at a temperature in the range from 30 to 60°C to a dry matter content of 10 to 20%, alcohol or acetone is added to the concentrate to precipitate the gelatinous substances responsible for the cloudy appearance of the beverage reconstituted from the powder, maltodextrin is added to the centrifuged water-alcohol or water-acetone mixture and the resulting mixture is dried to obtain an instant powder.

2. A process as claimed in claim 1, characterised in that three extractions with water are carried out.

3. A process as claimed in any of claims 1 or 2, characterised in that ethanol or acetone is added to the concentrate so that an alcohol or acetone content of 48 to 52% is obtained and the mixture is left standing for 8 to 20 hours at ambient temperature.

4. A process as claimed in any of claims 1 to 3, characterised in that drying is carried out in vacuo or by spray drying.

5. A process as claimed in claim 1, characterised in that the ground valerian roots are extracted three times at a temperature of 70°C, the aqueous extract is concentrated at a temperature of 48°C under a vacuum of 10⁴ Pa and then 2.10³ to obtain a dry matter content of approximately 14%, ethanol or acetone is added so that an alcohol or acetone content of 50% is obtained, the mixture is left standing at ambient temperature for 12 hours and then centrifuged, maltodextrin is added to the water-alcohol or water-acetone mixture in a quantity sufficient to give a ratio by weight of 1:1 of dry matter of the water-alcohol or water-acetone mixture to maltodextrin and the whole is dried at 50°C under a vacuum of the order of 10³ Pa.

## Patentansprüche

1. Verfahren zur Herstellung eines desodorisierten pulverförmigen Extraktes aus Baldrianwurzeln, dadurch gekennzeichnet, daß man die gemahlenen Baldrianwurzeln wenigstens einmal mit Wasser bei einer Temperatur von 65 bis 75°C während 2 bis 5 Stunden extrahiert, den wäßrigen Extrakt bei einem Druck von 2.10⁴ Pa bis 2.10³ Pa bei einer Temperatur von 30 bis 60°C auf einen Trockengehalt von 10 bis 20% konzentriert, dem Konzentrat Alkohol oder Azeton zusetzt, um die gelatinösen Substanzen auszufällen, die für das trübe Aussehen des aus dem Pulver rekonstituierten Getränks verantwortlich sind, dem zentrifugierten, wässrig-alkoholischen oder wässrig-azetonischen Gemisch Maltodextrin zusetzt und zu einem Instant-Pulver trocknet.

2. Verfahren nach Anspruch 1, dadurch gekennzeichnet, daß dreimal mit Wasser extrahiert wird.

3. Verfahren nach einem der Ansprüche 1 oder 2, dadurch gekennzeichnet, daß man dem Konzentrat Ethanol oder Azeton bis zu einem Gehalt an Alkohol oder an Azeton von 48 bis 52% zusetzt und anschließend bei Umgebungstemperatur 8 bis 20 Stunden ruhen läßt.

4. Verfahren nach einem der Ansprüche 1 bis 3, dadurch gekennzeichnet, daß man durch Sprühtrocknung oder im Vakuum trocknet.

5. Verfahren nach Anspruch 1, dadurch gekennzeichnet, daß man die gemahlenen Baldrianwurzeln dreimal bei einer Temperatur von 70°C extrahiert, den wäßrigen Extrakt bei einer Temperatur von 48°C unter einem Vakuum 10⁴ Pa und dann 2.10³ Pa bis zu einem Trockengehalt von etwa 14% konzentriert, Ethanol oder Azeton bis zu einem Alkohol- oder Azetongehalt von 50% zusetzt, bei Umgebungstemperatur 12 Stunden lang ruhen läßt, zentrifugiert, dem wässrig-alkoholischen oder wässrig-azetonischen Gemisch Maltodextrin bis zu einem Gewichtsverhältnis von Trockenmaterial des wässrig-alkoholischen oder wässrig-azetonischen Gemisches zu Maltodextrin von 1:1 zusetzt und bei 50°C unter einem Vakuum in der Größenordnung von 10³ Pa trocknet.
